# EUROPEAN PATENT SPECIFICATION

(11) **EP 4 178 489 B1**
(45) Date of publication and mention of the grant of the patent: **30.10.2024**
(21) Application number: 21749538.1
(22) Date of filing: 13.07.2021
(51) Int. Cl.: A61F 2/24

(54) **ANNULOPLASTY DEVICE**
ANNULOPLASTIEVORRICHTUNG
DISPOSITIF D'ANNULOPLASTIE

(30) Priority: 13.07.2020 US 202063051128 P
(43) Date of publication of application: 17.05.2023
(73) Proprietor: HVR Cardio Oy, 02600 Espoo (FI)
(72) Inventor: KERÄNEN, Olli, 23741 Bjärred (SE)
(74) Representative: Invent Horizon IP
(86) International application number: PCT/EP2021/069433
(87) International publication number: WO 2022/013207

(56) References cited:
- EP-A1- 2 611 387
- EP-A1- 2 661 239
- EP-A1- 3 766 457
- EP-B1- 2 611 387
- EP-B1- 2 661 239
- WO-A1-2019/081777

## Description

### Field of the Invention

This invention pertains in general to the field of cardiac valve repair. More particularly the invention relates to an annuloplasty device or implant, such as an annuloplasty ring or helix, for positioning at the heart valve annulus.

### Background of the Invention

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure. The annuloplasty ring is typically implanted around the annulus of the heart valve.

A problem with prior art annuloplasty implants is to achieve correct positioning at the heart valve and fixate the implant in the correct position. Suturing devices for annuloplasty implants have disadvantages that makes it difficult to suture in the correct position, thereby resulting insufficient suturing strength, and also in a very time-consuming procedure, which increases the risks for the patient. Furthermore, suturing devices are often not sufficiently compact for catheter based procedures. The use of clips for positioning annuloplasty implants is also associated with challenges, in particular when implanting helix rings that are to be positioned on either side of a heart valve. Insufficient fixation of such implant lead to traumatic effects since the fixation structure must ensure the correct position of the device over time. A further problem in the prior art is thus also to achieve a reliable fixation at the annulus of the heart valve. An annuloplasty implant is intended to function for years and years, so it is critical with long term stability in this regard.

WO2019/081777A1 discloses an annuloplasty device comprising first and second support rings being configured to be arranged as a coil in a first configuration around an axial direction. The first and second support rings are configured to be arranged on opposite sides of native heart valve leaflets of a heart valve. An anterior portion, and posterior bows are disclosed and the supports have fastening units.

The above problems may have dire consequences for the patient and the health care system. Patient risk is increased.

Hence, an improved annuloplasty implant would be advantageous and in particular allowing for avoiding more of the above mentioned problems and compromises, and in particular ensuring secure fixation of the annuloplasty implant, during the implantation phase, and for long-term functioning, in addition to a less complex procedure, and increased patient safety. A related method of manufacturing would also be advantageous.

### Summary of the disclosure

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect an annuloplasty device is provided comprising first and second support rings having a coiled configuration in which the first and second support rings are arranged as a coil around a central axis, wherein the first and second support rings are configured to be arranged on opposite sides of native heart valve leaflets of a heart valve, wherein the first and/or second support ring comprises a plurality of sides forming a non-circular cross-section of the first and/or second support ring, wherein the cross-section varies along a longitudinal direction of the first and/or second support ring.

According to a second aspect a method of manufacturing an annuloplasty device, comprising providing a sheet of a biocompatible metal alloy, cutting first and second support sections from the sheet, arranging the first and second support sections in a coiled configuration so that the first and second support sections form first and second support rings arranged as a coil around a central axis, heating the first and second support rings to heat-set the coiled configuration of the annuloplasty device.

According to a third aspect , not part of the invention, a method of repairing a defective heart valve is provided comprising positioning a second support ring of an annuloplasty device on a ventricular side of the heart valve, and positioning a first support ring of the annuloplasty device on an atrial side of the heart valve, the first and second support rings are arranged as a coil around a central axis on opposite sides of native heart valve leaflets of the heart valve, the first and/or second support ring comprises a plurality of sides forming a non-circular cross-section of the first and/or second support ring, wherein the cross-section varies along a longitudinal direction of the first and/or second support ring.

Further examples of the invention are defined in the dependent claims, wherein features for the second aspect are as for the first aspect mutatis mutandis.

Some examples of the disclosure provide for a facilitated positioning of an annuloplasty implant at a heart valve.

Some examples of the disclosure provide for a facilitated fixation of an annuloplasty implant at a heart valve.

Some examples of the disclosure provide for a less time-consuming fixation of an annuloplasty to a target site.

Some examples of the disclosure provide for securing long-term functioning and position of an annuloplasty implant.

Some examples of the disclosure provide for a more secure implantation of an annuloplasty implant in narrow anatomies.

Some examples of the disclosure provide for an annuloplasty device with an increased retention force at the heart valve.

Some examples of the disclosure provide for a facilitated manufacturing of an annuloplasty device.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1a is a schematic illustration, in a top-down view, of an annuloplasty implant or device with first and second support rings, according to an example;
Fig. 1b is a schematic illustration, in a side view, of an annuloplasty device with first and second support rings, according to an example;
Figs. 2a-b are schematic illustrations of cross-sections of an annuloplasty device, according examples of the disclosure;
Figs. 3a-b are schematic illustrations of cross-sections of an annuloplasty device, according examples of the disclosure;
Figs. 4a-b are schematic illustrations of cross-sections of an annuloplasty device, according examples of the disclosure;
Figs. 5a-b are schematic illustrations of cross-sections of an annuloplasty device, according examples of the disclosure;
Fig. 6 is a schematic illustration, in a perspective view, of an annuloplasty device with first and second support rings, according to an example;
Fig. 7 is a schematic illustration, in a side view, of an annuloplasty device positioned at a heart valve in a coiled configuration, according to an example;
Fig. 8a is a flow chart of a method of manufacturing an annuloplasty device, according to one example; and
Fig. 8b is a flow chart of a method of repairing a defective heart valve according to one example.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

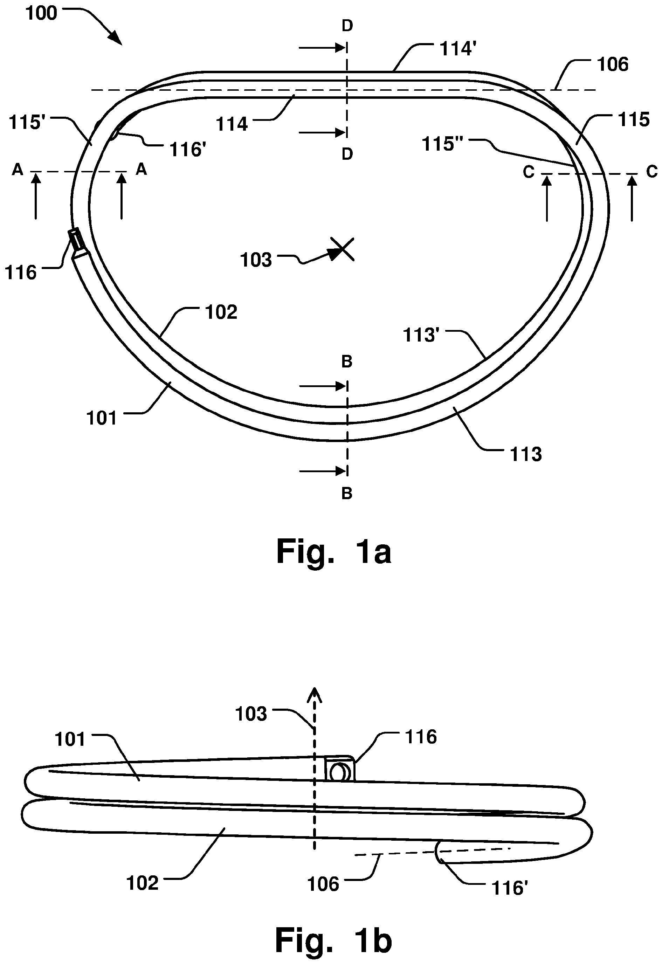

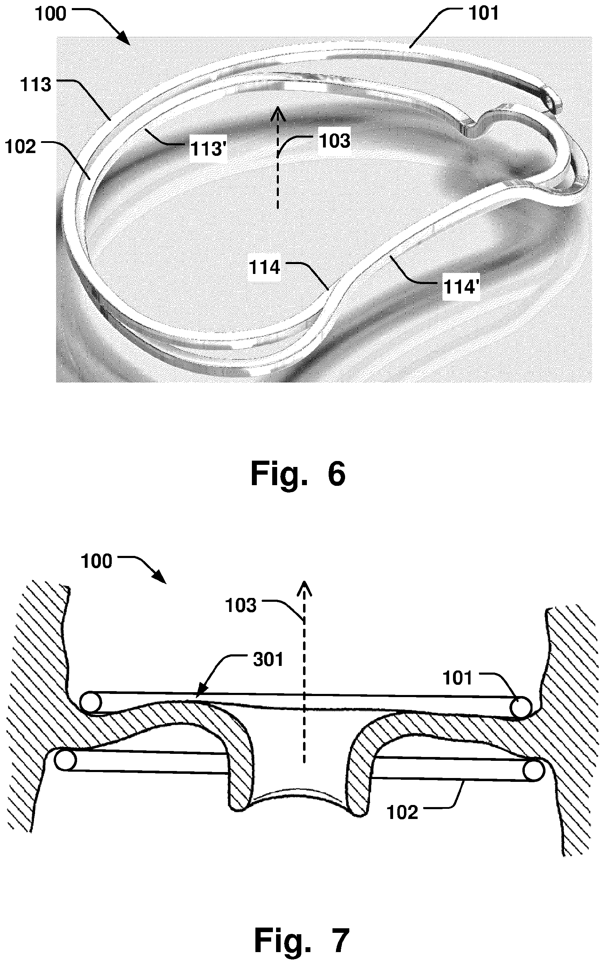

Fig. 1a schematically illustrates an example of an annuloplasty device 100 comprising a first support ring 101 and second support ring 102 which are adapted to be arranged as a coil, i.e. in a helix-shape, in a coiled configuration around a central axis 103, as illustrated in the top-down view of Fig. 1a and the side view of Fig. 1b. The device 100 is arranged in the coiled configuration at least when in a relaxed state of the material from which the device 100 is formed, i.e. free from outside forces acting upon the device 100. The coil-shaped device 100 has two free ends 116, 116'. The first and second support rings 101, 102, and the respective free ends 116, 116', are configured to be arranged on opposite sides of native heart valve leaflets 301 of a heart valve, as illustrated in e.g. the side view of Fig. 7. As shown in Fig. 7, the first support ring 101 may be arranged on an atrial side of the heart valve, and the second support ring 102 may be arranged on a ventricular side. The first support ring 101 may thus extend along the annulus of the heart valve on the atrial side. The first and second support rings 101, 102, are connected to form a coil- or helix shaped ring, as an integral continuous ring. The coil extends through the valve opening at a commissure thereof. The first and second support rings 101, 102, may thus assume the coiled configuration also when in an implanted state. As explained further below, the device 100 may comprise a shape-memory material, so that the device 100 re-assumes the coiled configuration after having been delivered from a catheter (not shown) to the target site, after having been temporarily restrained in an elongated configuration of the catheter. The annuloplasty device 100, i.e. annuloplasty implant 100, may comprise a shape memory material, such as NiTiNol, or another suitable biocompatible alloy that can be heat-set in defined shapes, i.e. in a defined relaxed shape in absence of outside acting forces, as described further below, in a heat treatment procedure. The annuloplasty device 100 may pinch the tissue of the valve leaflets 301, between the first and second support rings 101, 102, i.e. with forces acting parallel with the central axis 103.

The device 100 may comprise a shape-memory material, so that the first and second rings 101, 102, assumes the first configuration after having been ejected from a delivery catheter (not shown). While positioned in the delivery catheter the device 100 may be stretched in an elongated shape. Alternatively, the device 100 may be arranged in the coiled configuration when being delivered to the target site, in which case it may be implanted at the target site for example by incision between the ribs or by opening the chest. The present disclosure, and the associated advantages described for the various examples, applies to both such variants of the device 100.

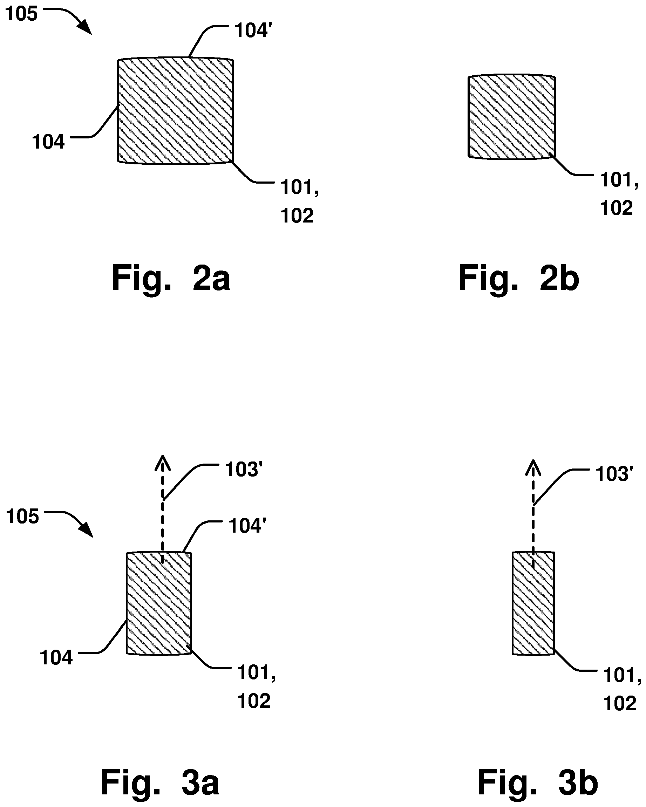

The first and/or second support ring 101, 102, comprises a plurality of sides 104, 104' forming a non-circular cross-section 105 of the first and/or second support ring 101, 102. Figs. 2a-b, 3a-b, 4a-b, and 5a-b show examples of such non-circular cross-sections 105. Different cross-section lines A-A, B-B, C-C, and D-D are indicated in Fig. 1a. The cross-section 105 of the first and/or second support ring 101, 102, along the aforementioned cross-section lines may correspond to any of the examples in Figs. 2a-b, 3a-b, 4a-b, and 5a-b.

The cross-section 105 varies along a longitudinal direction 106 of the first and/or second support ring 101, 102. Having a non-circular shape provides for increasing the compression force between the first and second rings 101, 102, in the coiled configuration while maintaining a compact cross-sectional profile of the first and second rings 101, 102. The dimensions of the sides 104, 104', may be varied in order to provide for an optimized bending resistance of the support rings 101, 102. Varying the aforementioned dimensions along the length of the first and second support rings 101, 102, i.e. along the longitudinal direction 106, allows for varying the flexibility of the rings 101, 102, along the longitudinal direction 106 and be customized to different anatomical positions around the annulus of the heart valve. This provides for better accommodating movement of the tissue which may be greater at localized sections of the annulus, while other sections may have an increased rigidity for a stronger pinching effect between the first and second support rings 101, 102. A more secure and robust positioning of the device 100 may thus be provided and improved long-term functioning. A varying cross-section 105 provides also for optimizing the flexibility with respect to the delivery and positioning phase of the annuloplasty device 100. E.g. portions of the first and second support rings 101, 102, which are subject to the most bending movement when being inserted in a delivery catheter (not shown) may have a cross-section which increases the flexibility. Those portions may be the commissure sections 115, 115', 115", as described further below. Increasing the flexibility of those sections allows for reducing the force needed to advance the annuloplasty device 100 in the delivery catheter and facilitating the deployment of the annuloplasty device 100 at the target site. The implantation procedure is thus facilitated. Other portions of the first and second support rings 101, 102, such as the posterior bows 113, 113', may simultaneously have a different cross-section which provides for a decreased flexibility and a greater pinching force between the rings 101, 102, in the axial direction 103. Fig. 6 shows a perspective view of an annuloplasty device 100 comprising first and second support rings 101, 102. The first and/or second support ring 101, 102, may have a varying non-circular cross-section 105 as described above, providing for the aforementioned advantageous benefits.

The cross-section 105 may be essentially rectangular, as shown in the example of Figs. 2 - 5. This may provide for particularly advantageous mechanical characteristics for increasing the compression force between the first and second support rings 101, 102, while maintaining a compact cross-section. The length of the sides 104, 104', may vary as exemplified in Figs. 2 - 5, and it is conceivable that the length of the sides 104, 104', are varied for optimization to different applications, and also varied along the length of the support rings 101, 102, as elucidated above. It should be understood that the cross-sections 105 in Figs. 2-5 are examples and that other variations of the cross-section 105 of the first and/or second support ring 101, 102, are conceivable.

The area of the cross-section 105 varies along the longitudinal direction 106. Figs. 2a-b show an example of a varying area of the cross-section 105, where the area of the cross-section 105 in Fig. 2b is less compared to the areas of the cross-section 105 in Fig. 2a. The first and/or second support ring 101, 102, may have two different cross-sections 105, such as exemplified in Figs. 2a-b, along different cross-sectional lines A-A, B-B, C-C, D-D as exemplified in Fig. 1a. For example, the area of the cross-section 105 along A-A or C-C may be less than the area of the cross-section 105 along B-B or D-D. This may provide for increased flexibility along A-A and/or C-C, corresponding to commissure sections 115', 115, compared to the sections at B-B and/or D-D, corresponding to posterior bows 113, 113'. This may provide for the advantageous benefits as described above. The transition between the different areas is gradual along the longitudinal direction 106.

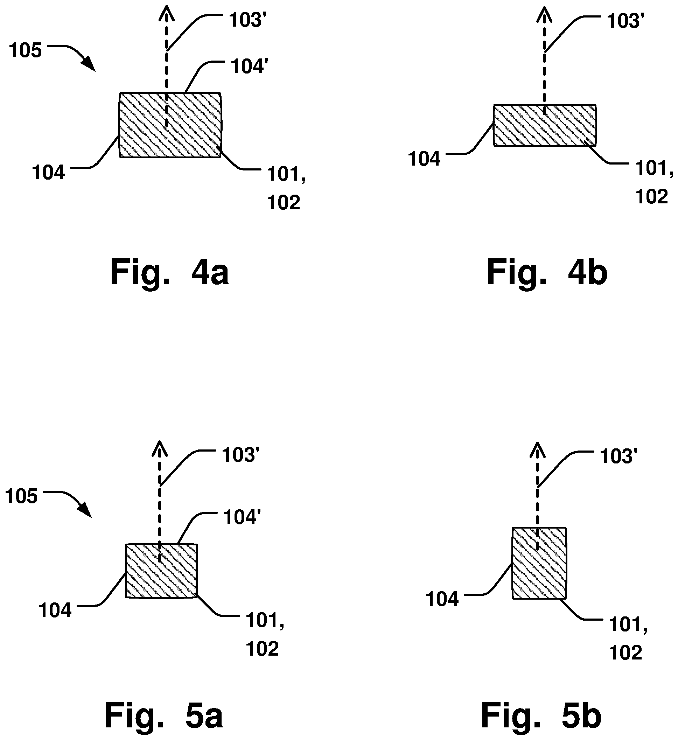

The shape of the cross-section 105 may vary along the longitudinal direction 106. Figs. 3-5 are schematic examples of such varying shape. Turning to e.g. Figs. 5a-b, the length of the sides 104 extending in direction 103' being parallel with the central axis 103 may be varied. E.g. Fig. 5b show longer sides 104 in the aforementioned direction compared to the cross-section in Fig. 5a. Increasing the dimension of the sides 104 to create a taller profile may provide for increasing the bending resistance along the axial direction 103, compared to the thinner profile in Fig. 5a in this direction. The area of the cross-sections 105 in Figs. 5a-b may be similar or the same, but the varying the shape allows for tailoring the bending resistance while maintaining the overall structural rigidity provided by the unchanged cross-sectional area. Different portions of the first and/or second support rings 101, 102, may thus be optimized according to their intended positions along the heart valve annulus and in what direction the forces should act to provide a strong retention force but also compliance to the anatomy and the dynamic motions in the heart. The first and/or second support ring 101, 102, may have two different cross-sections 105, such as exemplified in Figs. 5a-b, along different cross-sectional lines A-A, B-B, C-C, D-D as exemplified in Fig. 1a.

Turning to Fig. 1a, the first support ring 101 may comprise a first posterior bow 113 and a first anterior portion 114. The second support ring 102 may comprise a second posterior bow 113' and a second anterior portion 114'. The first and second posterior bows 113, 113', are adapted to conform to a posterior aspect of said heart valve, and the first and second anterior portions 114, 114', are adapted to conform to an anterior aspect of said heart valve. The first posterior bow 113 transitions to the first anterior portion 114 over a first commissure section 115. The first anterior portion 114 transitions to the second posterior bow 113' over a second commissure section 115'. The second posterior bow 113' transitions to the second anterior portion 114' over a third commissure section 115".

Any of the first, second and third commissure sections 115, 115', 115" may have a cross-section which has an area and/or shape which is different than an area and/or shape of the cross-section 105 of any of the first posterior bow 113, the second posterior bow 113', the first anterior portion 114, and the second anterior portion 114'. The bending radius of the first, second and third commissure sections 115, 115', 115", is less than the bending radius of any of the first posterior bow 113, the second posterior bow 113', the first anterior portion 114, and the second anterior portion 114'. Having a different area and/or shape of the cross-section 105 at the first, second and/or third commissure section 115, 115', 115", compared to the aforementioned portions may thus provide for optimizing the dynamic behavior of the annuloplasty device 100 at 115, 115', 115". E.g. to facilitate stretching the annuloplasty device 100 in a delivery catheter or positioning the annuloplasty device 100 at opposite sides of the heart valve, where the first and second support rings 101, 102, gradually assume the defined heat-set coiled configuration upon ejection from the delivery catheter. Also, the pinching force may be optimized to the particular application, since the first commissure sections 115, in the example of Fig. 1a, is also the transition point at the commissure connecting the first and second support rings 101, 102, through the heart valve opening. The area and/or shape of the first commissure sections 115 may thus be varied to provide such pinching force between the rings 101, 102. E.g. in one example, the area and/or shape of the cross-section 105 at the second and third commissure sections 115', 115", may provide for increased flexibility, for facilitated deployment through a catheter, compared to the first commissure section 115, which may instead be optimized for increasing the pinching effect between the rings 101, 102, in the axial direction 103 (Fig. 7)

In one example, any of the first, second and third commissure sections 115, 115', 115" may have a cross-section 105 which has an area which is less than an area of the cross-section 105 of any of the first posterior bow 113, the second posterior bow 113', the first anterior portion 114, and the second anterior portion 114'. This may provide for increasing the flexibility at any of the first, second and third commissure sections 115, 115', 115", compared to the aforementioned portions, to provide for the advantageous benefits as mentioned above.

The first and/or second anterior portion 114, 114', may have a cross-section 105 which has an area and/or shape which is different than an area and/or shape of the cross-section 105 of the first and/or second posterior bow 113, 113'. The pinching force in the axial direction 103 and the flexibility of the annuloplasty device 100 during delivery and implantation may thus be optimized by tailoring the cross-section along the anterior portions 114, 114', and the posterior bows 113, 113'. In one example, it may be desirable to have a greater pinching force between the rings 101, 102, along the posterior bows 113, 113', compared to the anterior portions 114, 114'. E.g. the tissue may in some cases be more sensitive along the anterior portions 114, 114', and a greater flexibility along the latter portions may thus be desirable. As elucidated above, the flexibility may be increased by reducing the length of the sides 104 extending parallel with the central axis, and/or by generally reducing the area of the cross-section 105.

The first anterior portion 114 may have a cross-section 105 which has an area and/or shape which is different than an area and/or shape of the cross-section 105 of the second anterior portion 114'. The anatomy and the dynamic characteristics of the heart valve motions may be different on the atrial and ventricular sides of the heart valve. It may thus be advantageous to have different cross-sections 105 on first and second anterior portions 114, 114', to provide the desired compliance to the anatomy while having the rigidity to exert a compressive force which anchors the annuloplasty device 100 in place.

Similarly, the first posterior bow 113 may have a cross-section 105 which has an area and/or shape which is different than an area and/or shape of the cross-section 105 of the second posterior bow 113', to accommodate variations in the anatomy on the ventricular and atrial sides.

The cross-section 105 may be elongated in a direction 103' extending essentially in parallel with the central axis 103. This provides for increasing the compression force in a direction parallel with the central axis 103, while allowing for increasing the flexibility in a direction perpendicular to the central axis 103. Having an increased flexibility perpendicular to the central axis 103 may facilitate bending the first and second support rings 101, 102, to an elongated straight configuration for insertion in a delivery catheter. Hence, such elongated "band-profile" as exemplified in Fig. 3a or 3b, may provide for increasing the compression force while also allowing for a facilitated implantation via a delivery catheter (not shown). The elongated profile may vary, as exemplified in Figs. 3a-b, to adapt the flexibility and retention as described above. Thus, the first and/or second support ring 101, 102, may have two different cross-sections 105, such as exemplified in Figs. 3a-b, along different cross-sectional lines A-A, B-B, C-C, D-D as exemplified in Fig. 1a.

Thus, the cross-section 105 may be rectangular so that the longest sides 104 of the rectangular shape extend essentially in parallel with the central axis 103.

Figs. 4a-b show another example where the cross-section 105 is elongated but the short side 104 of the rectangle is perpendicular to the direction 103'. Direction 103' is parallel with the central axis 103. Such alignment of the short side 104 provides for increased flexibility for bending in the axial direction 103, which may be desirable in some applications. The area and/or shape of the cross-section 105 may vary as illustrated in Figs. 4a-b, while maintaining the same alignment of the generally elongated shape of the cross-section 105. E.g. the flexibility along the central axis 103 may be increased even further at some portions of the annuloplasty device 100 by reducing the height of the sides 104 as schematically indicated in Fig. 4b, compared to Fig. 4a. The first and/or second support ring 101, 102, may have two different cross-sections 105, such as exemplified in Figs. 4a-b, along different cross-sectional lines A-A, B-B, C-C, D-D as exemplified in Fig. 1a.

The first support ring 101 may have a cross-section 105 which has an area and/or shape which is different than an area and/or shape of the cross-section 105 of the second support ring 102. The variation between the first and second rings 101, 102, may be exemplified by any of the above discussed variations.

The first and second support rings 101, 102, may be formed from a shape-memory material. The first and second support rings 101, 102, may have an elongated delivery configuration for advancement in a catheter, as described above. The first and second support rings 101, 102, may thus assume the coiled configuration again, after being ejected from a delivery catheter.

Fig. 6 shows a perspective view of an annuloplasty device 100 comprising first and second support rings 101, 102. The first and/or second support ring 101, 102, may have a varying non-circular cross-section 105 as described above in relation to Figs. 1 - 5, thus providing for the aforementioned advantageous benefits.

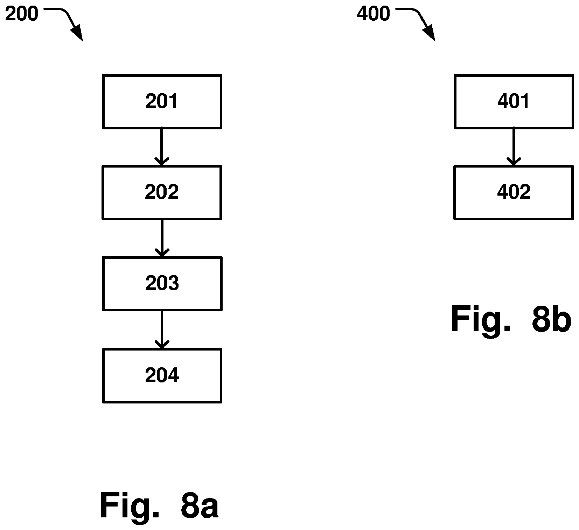

A method 200 of manufacturing an annuloplasty device is provided. The method 200 is schematically illustrated in Fig. 8a, in conjunction with Figs. 1 - 7. The order in which the steps are described should not be construed as limiting, and it is conceivable that the order of the steps may be varied depending on the particular procedure. The method 200 comprises providing 201 a sheet (not shown) of a biocompatible metal alloy. The method 200 comprises cutting 202 first and second support sections (not shown) from the sheet. The method 200 comprises arranging 203 the first and second support sections in a coiled configuration so that the first and second support sections form first and second support rings 101, 102, arranged as a coil around a central axis 103, as schematically illustrated in Fig. 1a. The method 200 comprises heating 204 the first and second support rings 101, 102, to heat-set the coiled configuration of the annuloplasty device 100. This provides for an effective, cost-efficient and facilitated manufacturing method of the annuloplasty device 100. The cutting of the support rings 101, 102 (to be formed) from the sheet may be provided by laser cutting, punching, or any other method for separating the desired support sections from the sheet.

A method 400 of repairing a defective heart valve is provided. The method 400 is schematically illustrated in Fig. 8b, in conjunction with Figs. 1 - 7. The order in which the steps are described should not be construed as limiting, and it is conceivable that the order of the steps may be varied depending on the particular procedure. The method 400 comprises positioning 401 a second support ring 102 of an annuloplasty device 100 on a ventricular side of the heart valve. The method 400 comprises positioning 402 a first support ring 101 of the annuloplasty device 100 on an atrial side of the heart valve. The first and second support rings are arranged as a coil around a central axis 103 on opposite sides of native heart valve leaflets of the heart valve. The first and/or second support ring 101, 102, comprises a plurality of sides 104, 104' forming a non-circular cross-section 105 of the first and/or second support ring 101, 102. The cross-section 105 varies along a longitudinal direction 106 of the first and/or second support ring 101, 102. The method 400 thus provides for the advantageous benefits as described above with respect to the annuloplasty device 100 and Figs. 1 - 7.

The first 101 and/or second support 102 may comprise a shape memory material, such as NiTiNol, or another suitable biocompatible alloy that can be heat-set in defined shapes, in a heat treatment procedure. The shape-memory material may comprise a material having more than one phase, so that the shape of the support rings 101, 102, may be actively varied as described above. The shape memory material can be conceived as any material that is able to change shape as desired, in response to outside interaction, for example with an energy source, such as providing heat and/or electromagnetic energy, that can be transferred to the device 100 to change its shape. It is also conceivable that the shape of the device 100 can be affected by direct mechanical manipulation of the curvature of the first 101 and/or second support 102, e.g. by transferring a force or torque to the device 100 via a delivery device. Via the various mentioned shape-affecting procedures the device 100 may assume an elongated delivery configuration for advancement in a catheter, an initial shape when positioned in a coiled configuration along the annulus of the valve, i.e. the first configuration, and also an activated shape such as the contracted state described above for enhancing the strength of the fixation at an annulus of the heart valve.

The support rings 101, 102, may be formed from a solid rod or other solid elongated structure, having various cross-sections, such as circular, elliptic, rhombic, triangular, rectangular etc. The support rings 101, 102, may be formed from a hollow tube, or other hollow structures with the mentioned cross-sections. The support rings 101, 102, may be formed from a sandwiched laminate material, comprising several layers of different materials, or different layers of the same material. The support rings 101, 102, may be formed from a stent or a stent-like structure, and/or a braided material. The support rings 101, 102, may be formed from a braid of different materials braided together, or from a braid of the same material. As mentioned, the support rings 101, 102, may be formed from NiTinol, or another suitable bio-compatible material. The surfaces of the first and second support rings 101, 102, may be provided with other materials and/or treated with different materials and/or structured to enhance resistance to breaking in case the material is repeatedly bent.

The first and second support rings 101, 102, may have an elongated delivery configuration for advancement in a catheter, and an implanted shape in the above described contracted state.

The present invention has been described above with reference to specific embodiments. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications .

## Claims

1. An annuloplasty device (100) comprising
first (101) and second (102) support rings having a coiled configuration in which the first and second support rings are arranged as a coil around a central axis (103), wherein the first and second support rings are configured to be arranged on opposite sides of native heart valve leaflets (301) of a heart valve,
wherein the first and/or second support ring comprises a plurality of sides (104, 104') forming a non-circular cross-section (105) of the first and/or second support ring, wherein the cross-section varies along a longitudinal direction (106) of the first and/or second support ring,
wherein the first support ring comprises a first posterior bow (113) and a first anterior portion (114),
the second support ring comprises a second posterior bow (113') and a second anterior portion (114'),
the first and second posterior bows are adapted to conform to a posterior aspect of said heart valve, and the first and second anterior portions are adapted to conform to an anterior aspect of said heart valve,
the first posterior bow transitions to the first anterior portion over a first commissure section (115),
the first anterior portion transitions to the second posterior bow over a second commissure section (115'),
the second posterior bow transitions to the second anterior portion over a third commissure section (115"),
wherein any of the first, second and third commissure sections (115, 115', 115") have a first cross-section which has an area and/or shape which is different than an area and/or shape of a second cross-section of any of the first posterior bow (113), the second posterior bow (113'), the first anterior portion (114), and the second anterior portion (114'), wherein the first cross-section transitions gradually to the second cross-section along the longitudinal direction.

2. Annuloplasty device according to claim 1, wherein the cross-section is essentially rectangular.

3. Annuloplasty device according to claim 1 or 2, wherein the area of the cross-section varies along the longitudinal direction.

4. Annuloplasty device according to any of claims 1 - 3, wherein the shape of the cross-section varies along the longitudinal direction.

5. Annuloplasty device according to claim 1, wherein any of the first, second and third commissure sections (115, 115', 115") have a cross-section which has an area which is less than an area of the cross-section of any of the first posterior bow (113), the second posterior bow (113'), the first anterior portion (114), and the second anterior portion (114').

6. Annuloplasty device according to any of claims 4 - 5, wherein the first and/or second anterior portion (114, 114') has a cross-section which has an area and/or shape which is different than an area and/or shape of the cross-section of the first and/or second posterior bow (113, 113').

7. Annuloplasty device according to any of claims 4 - 6, wherein the first anterior portion (114) has a cross-section which has an area and/or shape which is different than an area and/or shape of the cross-section of the second anterior portion (114').

8. Annuloplasty device according to any of claims 4 - 7, wherein the first posterior bow (113) has a cross-section which has an area and/or shape which is different than an area and/or shape of the cross-section of the second posterior bow (113').

9. Annuloplasty device according to any of claims 1 - 8, wherein the cross-section is elongated in a direction (103') extending essentially in parallel with the central axis.

10. Annuloplasty device according to claim 9, wherein the cross-section is rectangular so that the longest sides (104) of the rectangular shape extend essentially in parallel with the central axis.

11. Annuloplasty device according to any of claims 1 - 10, wherein the first support ring has a cross-section which has an area and/or shape which is different than an area and/or shape of the cross-section of the second support ring.

12. Annuloplasty device according to any of claims 1 - 11, wherein the first and second support rings are formed from a shape-memory material, wherein the first and second support rings have an elongated delivery configuration for advancement in a catheter.

13. Method (200) of manufacturing the annuloplasty device of claims 1 - 12 comprising
providing (201) a sheet of a biocompatible metal alloy,
cutting (202) first and second support sections from the sheet,
arranging (203) the first and second support sections in a coiled configuration so that the first and second support sections form the first and second support rings (101, 102) arranged as a coil around the central axis (103),
heating (204) the first and second support rings to heat-set the coiled configuration of the annuloplasty device.

## Patentansprüche

1. Annuloplastievorrichtung (100), umfassend
einen ersten (101) und einen zweiten (102) Stützring mit einer gewundenen Auslegung, in der der erste und der zweite Stützring als eine Windung um eine Mittelachse (103) angeordnet sind, wobei der erste und der zweite Stützring dazu ausgelegt sind, auf gegenüberliegenden Seiten der natürlichen Herzklappensegel (301) einer Herzklappe angeordnet zu werden,
wobei der erste und/oder der zweite Stützring eine Vielzahl von Seiten (104, 104') umfasst, die einen nicht kreisförmigen Querschnitt (105) des ersten und/oder zweiten Stützrings bilden, wobei der Querschnitt entlang einer Längsrichtung (106) des ersten und/oder zweiten Stützrings variiert,
wobei der erste Stützring einen ersten posterioren Bogen (113) und einen ersten anterioren Abschnitt (114) umfasst,
der zweite Stützring einen zweiten posterioren Bogen (113') und einen zweiten anterioren Abschnitt (114') umfasst,
der erste und der zweite posteriore Bogen dazu angepasst sind, einem posterioren Aspekt der Herzklappe zu entsprechen, und der erste und der zweite anteriore Abschnitt dazu angepasst sind, einem anterioren Aspekt der Herzklappe zu entsprechen,
der erste posteriore Bogen über einem ersten Kommissurabschnitt (115) in den ersten anterioren Abschnitt übergeht,
der erste anteriore Bogen über einem zweiten Kommissurabschnitt (115') in den zweiten anterioren Abschnitt übergeht,
der zweite posteriore Bogen über einem dritten Kommissurabschnitt (115") in den zweiten anterioren Abschnitt übergeht,
wobei einer des ersten, zweiten und dritten Kommissurabschnitts (115, 115', 115") einen ersten Querschnitt hat, der eine Fläche und/oder Form hat, die sich von einer Fläche und/oder Form eines zweiten Querschnitts von einem des ersten posterioren Bogens (113), des zweiten posterioren Bogens (113'), des ersten anterioren Abschnitts (114) und des zweiten anterioren Abschnitts (114') unterscheidet, wobei der erste Querschnitt entlang der Längsrichtung allmählich in den zweiten Querschnitt übergeht.

2. Annuloplastievorrichtung nach Anspruch 1, wobei der Querschnitt im Wesentlichen rechteckig ist.

3. Annuloplastievorrichtung nach Anspruch 1 oder 2, wobei die Fläche des Querschnitts entlang der Längsrichtung variiert.

4. Annuloplastievorrichtung nach einem der Ansprüche 1 - 3, wobei die Form des Querschnitts entlang der Längsrichtung variiert.

5. Annuloplastievorrichtung nach Anspruch 1, wobei einer des ersten, zweiten und dritten Kommissurabschnitts (115, 115', 115") einen Querschnitt hat, der eine Fläche hat, die kleiner ist als eine Fläche des Querschnitts von einem des ersten posterioren Bogens (113), des zweiten posterioren Bogens (113'), des ersten anterioren Abschnitts (114) und des zweiten anterioren Abschnitts (114').

6. Annuloplastievorrichtung nach einem der Ansprüche 4 - 5, wobei der erste und/oder zweite anteriore Abschnitt (114, 114') einen Querschnitt hat, der eine Fläche und/oder Form hat, die sich von einer Fläche und/oder Form des Querschnitts des ersten und/oder zweiten posterioren Bogens (113, 113') unterscheidet.

7. Annuloplastievorrichtung nach einem der Ansprüche 4 - 6, wobei der erste anteriore Abschnitt (114) einen Querschnitt hat, der eine Fläche und/oder Form hat, die sich von einer Fläche und/oder Form des Querschnitts des zweiten anterioren Bogens (114') unterscheidet.

8. Annuloplastievorrichtung nach einem der Ansprüche 4 - 7, wobei der erste und/oder zweite posteriore Abschnitt (113) einen Querschnitt hat, der eine Fläche und/oder Form hat, die sich von einer Fläche und/oder Form des Querschnitts des zweiten posterioren Bogens (113') unterscheidet.

9. Annuloplastievorrichtung nach einem der Ansprüche 1 - 8, wobei der Querschnitt in eine Richtung (103') verlängert ist, die sich im Wesentlichen parallel zu der Mittelachse erstreckt.

10. Annuloplastievorrichtung nach Anspruch 9, wobei der Querschnitt rechteckig ist, sodass sich die längsten Seiten (104) der rechtwinkligen Form im Wesentlichen parallel zu der Längsachse erstrecken.

11. Annuloplastievorrichtung nach einem der Ansprüche 1 - 10, wobei der erste Stützring einen Querschnitt hat, der eine Fläche und/oder Form hat, die sich von einer Fläche und/oder Form des Querschnitts des zweiten Stützrings unterscheidet.

12. Annuloplastievorrichtung nach einem der Ansprüche 1 - 11, wobei der erste und der zweite Stützring aus einem Formgedächtnismaterial gebildet sind, wobei der erste und der zweite Stützring eine längliche Zufuhrauslegung zur Vorwärtsbewegung in einem Katheter haben.

13. Verfahren (200) zur Herstellung der Annuloplastievorrichtung nach den Ansprüchen 1 - 12, umfassend
Bereitstellen (201) eines Blechs einer biokompatiblen Metalllegierung,
Schneiden (202) des ersten und des zweiten Stützbereiches aus dem Blech,
Anordnen (203) des ersten und des zweiten Stützbereiches in einer gewundenen Auslegung, sodass der erste und der zweite Stützbereich den ersten und den zweiten Stützring (101, 102) bilden, die als eine Windung um die Mittelachse (103) angeordnet sind,
Erhitzen (204) des ersten und des zweiten Stützrings, um die gewundene Auslegung der Annuloplastievorrichtung zu thermofixieren.

## Revendications

1. Dispositif d'annuloplastie (100) comprenant
des premier (101) et deuxième (102) anneaux de support ayant une configuration en spirale dans laquelle les premier et deuxième anneaux de support sont disposés comme une spirale autour d'un axe central (103), les premier et deuxième anneaux de support étant configurés pour être disposés sur des côtés opposés de feuillets de valve cardiaque naturels (301) d'une valve cardiaque,
dans lequel le premier et/ou le deuxième anneau de support comprennent une pluralité de côtés (104, 104') formant une section transversale non circulaire (105) du premier et/ou du deuxième anneau de support, la section transversale variant le long d'une direction longitudinale (106) du premier et/ou du deuxième anneau de support,
dans lequel le premier anneau de support comprend un premier arc postérieur (113) et une première partie antérieure (114),
le deuxième anneau de support comprend un deuxième arc postérieur (113') et une deuxième partie antérieure (114'),
les premier et deuxième arcs postérieurs sont adaptés pour épouser la forme d'une apparence postérieure de ladite valve cardiaque, et les première et deuxième parties antérieures sont adaptées pour épouser la forme d'une apparence antérieure de ladite valve cardiaque,
le premier arc postérieur rejoint la première partie antérieure sur une première section de commissure (115),
la première partie antérieure rejoint le deuxième arc postérieur sur une deuxième section de commissure (115'),
le deuxième arc postérieur rejoint la deuxième partie antérieure sur une troisième section de commissure (115"),
dans lequel n'importe laquelle des première, deuxième et troisième sections de commissure (115, 115, 115") a une première section transversale qui a une aire et/ou une forme qui sont différentes d'une aire et/ou d'une forme d'une deuxième section transversale de n'importe quel élément parmi le premier arc postérieur (113), le deuxième arc postérieur (113'), la première partie antérieure (114) et la deuxième partie antérieure (114'), la première section transversale passant progressivement à la deuxième section transversale le long de la direction longitudinale.

2. Dispositif d'annuloplastie selon la revendication 1, dans lequel la section transversale est essentiellement rectangulaire.

3. Dispositif d'annuloplastie selon la revendication 1 ou 2, dans lequel l'aire de la section transversale varie le long de la direction longitudinale.

4. Dispositif d'annuloplastie selon l'une quelconque des revendications 1 à 3, dans lequel la forme de la section transversale varie le long de la direction longitudinale.

5. Dispositif d'annuloplastie selon la revendication 1, dans lequel n'importe laquelle des première, deuxième et troisième sections de commissure (115, 115, 115") a une section transversale qui a une aire qui est inférieure à une aire de la section transversale de n'importe quel élément parmi le premier arc postérieur (113), le deuxième arc postérieur (113'), la première partie antérieure (114) et la deuxième partie antérieure (114').

6. Dispositif d'annuloplastie selon l'une quelconque des revendications 4 et 5, dans lequel la première et/ou la deuxième partie antérieure (114, 114') ont une section transversale qui a une aire et/ou une forme qui sont différentes d'une aire et/ou d'une forme de la section transversale du premier et/ou du deuxième arc postérieur (113, 113').

7. Dispositif d'annuloplastie selon l'une quelconque des revendications 4 à 6, dans lequel la première partie antérieure (114) a une section transversale qui a une aire et/ou une forme qui sont différentes d'une aire et/ou d'une forme de la section transversale de la deuxième partie postérieure (114').

8. Dispositif d'annuloplastie selon l'une quelconque des revendications 4 à 7, dans lequel le premier arc postérieur (113) a une section transversale qui a une aire et/ou une forme qui sont différentes d'une aire et/ou d'une forme de la section transversale du deuxième arc postérieur (113').

9. Dispositif d'annuloplastie selon l'une quelconque des revendications 1 à 8, dans lequel la section transversale est allongée dans une direction (103') s'étendant de façon essentiellement parallèle à l'axe central.

10. Dispositif d'annuloplastie selon la revendication 9, dans lequel la section transversale est rectangulaire de telle sorte que les côtés les plus longs (104) de la forme rectangulaire s'étendent de façon essentiellement parallèle à l'axe central.

11. Dispositif d'annuloplastie selon l'une quelconque des revendications 1 à 10, dans lequel le premier anneau de support a une section transversale qui a une aire et/ou une forme qui sont différentes d'une aire et/ou d'une forme de la section transversale du deuxième anneau de support.

12. Dispositif d'annuloplastie selon l'une quelconque des revendications 1 à 11, dans lequel les premier et deuxième anneaux de support sont formés à partir d'un matériau à mémoire de forme, les premier et deuxième anneaux de support ayant une configuration de délivrance allongée pour les faire avancer dans un cathéter.

13. Procédé (200) de fabrication du dispositif d'annuloplastie des revendications 1 à 12 comprenant
l'obtention (201) d'une feuille d'un alliage métallique biocompatible,
la découpe (202) de première et deuxième sections de support dans la feuille,
l'agencement (203) des première et deuxième sections de support dans une configuration en spirale de telle sorte que les première et deuxième sections de support forment les premier et deuxième anneaux de support (101, 102) disposés comme une spirale autour de l'axe central (103) ,
le chauffage (204) des premier et deuxième anneaux de support pour thermodurcir la configuration en spirale du dispositif d'annuloplastie.
